# EUROPÄISCHE PATENTSCHRIFT

(11) **EP 3 386 794 B1**
(45) Veröffentlichungstag und Bekanntmachung des Hinweises auf die Patenterteilung: **17.01.2024**
(21) Anmeldenummer: 16815738.6
(22) Anmeldetag: 28.11.2016
(51) Int. Cl.: B60K 28/06, A61B 5/00, B60W 40/08, A61B 5/07, A61M 5/142, A61N 1/372, G16H 40/60, G16H 40/63

(54) **VERFAHREN ZUR STEUERUNG EINER FUNKTIONALEN KOMPONENTE EINES KRAFTFAHRZEUGS MITTELS EINES MEDIZINISCHEN IMPLANTATS EINES BENUTZERS DES KRAFTFAHRZEUGS, KONTROLLVORRICHTUNG FÜR EIN KRAFTFAHRZEUG UND MEDIZINISCHES IMPLANTAT**
METHOD FOR CONTROLLING A FUNCTIONAL COMPONENT OF A MOTOR VEHICLE BY MEANS OF A MEDICAL IMPLANT OF A USER OF THE MOTOR VEHICLE, CONTROL DEVICE FOR A MOTOR VEHICLE, AND MEDICAL IMPLANT
PROCÉDÉ DE COMMANDE D'UN ÉLÉMENT FONCTIONNEL D'UN VÉHICULE AUTOMOBILE AU MOYEN D'UN IMPLANT MÉDICAL D'UN UTILISATEUR DU VÉHICULE AUTOMOBILE, DISPOSITIF DE CONTRÔLE D'UN VÉHICULE AUTOMOBILE ET IMPLANT MÉDICAL

(30) Priorität: 10.12.2015 DE 102015224836
(43) Veröffentlichungstag der Anmeldung: 17.10.2018
(73) Patentinhaber: Volkswagen Aktiengesellschaft, 38440 Wolfsburg (DE)
(72) Erfinder: BARTELS, Bastian, 38442 Wolfsburg (DE)
(86) Internationale Anmeldenummer: PCT/EP2016/079017
(87) Internationale Veröffentlichungsnummer: WO 2017/097624

(56) Entgegenhaltungen:
- EP-A1- 2 143 468
- EP-A1- 2 380 627
- DE-A1-102010 003 191
- DE-A1-102011 016 776
- DE-A1-102012 002 762
- US-A1- 2005 137 753
- US-A1- 2008 097 550
- US-A1- 2009 073 991
- US-A1- 2014 172 229

## Beschreibung

Die vorliegende Erfindung liegt auf dem Gebiet der Steuerung einer funktionalen Komponente eines Kraftfahrzeugs mittels eines medizinischen Implantats eines Benutzers des Kraftfahrzeugs.

Im medizinischen Bereich werden Patienten häufig mit Implantaten ausgestattet, z. B. Herzschrittmachern oder Kardioverter-Defibrillatoren, welche in der Lage sind, ein gesundheitliches Leiden des Patienten mit Präventivmaßnahmen zu lindern oder abzustellen. Verschlechtert sich jedoch der Gesundheitszustand des Patienten spontan über ein übliches Maß hinaus, kann für den Patienten eine unangenehme oder sogar kritische Situation entstehen, die beispielsweise im Straßenverkehr hinderlich sein kann. Konventionelle Fahrerassistenzsysteme können daher eine Teilsteuerung eines von dem Patienten geführten Fahrzeuges in einem solchen medizinischen Notfall übernehmen. Wenn das Auto keine Lenkeingriffe feststellt, obwohl die Sensoren des Spurhalteassistenten ein Abweichen von der Fahrbahn detektieren, kann z. B. der Fahrer gewarnt und schließlich das Auto kontrolliert zum Stillstand gebracht werden. Tritt dieser Fall jedoch beispielsweise bei Patienten mit implantierten Kardioverter-Defibrillatoren oder Herzschrittmachern ein, kann eine vermeidbare Verzögerung zwischen der Detektion des medizinischen Notfalls durch das Implantat einerseits und durch das Fahrzeug selbst andererseits entstehen. Die Zeit der Verzögerung kann möglicherweise für den Patienten körperlich unangenehm sein, oder auch zu einer unnötigen Behinderung weiterer Verkehrsteilnehmer führen. Derartige Probleme können jenseits des Straßenverkehrs auch in anderen Situationen auftreten, bei denen Maschinen von Implantatträgern bedient oder geführt werden.

Die Druckschrift DE 10 2012 002 762 A1 betrifft die Realisierung wenigstens einer vorbeugenden Maßnahme im Kraftfahrzeug als Reaktion auf empfangene Informationen über eine derzeitige oder bevorstehende Beeinträchtigung eines Führers des Kraftfahrzeugs.

Druckschrift EP 2 143 468 A1 betrifft zudem noch ein implantierbares medizinisches Gerät mit einer fernaktivierbaren Funkschnittstelle.

Mithin ist es wünschenswert, ein Konzept für eine Kommunikation zwischen einem medizinischen Implantat und einem Kraftfahrzeug zu verbessern.

Diesem Bedarf tragen eine Vorrichtung, ein Verfahren und ein Implantat mit den Merkmalen der unabhängigen Patentansprüche Rechnung. Weitere vorteilhafte Ausführungsformen und Weiterbildungen sind Gegenstand der abhängigen Patentansprüche.

Gemäß einem ersten Aspekt bezieht sich die Erfindung auf ein Verfahren zur Steuerung einer funktionalen Komponente eines Kraftfahrzeugs mittels eines medizinischen Implantats eines Benutzers des Kraftfahrzeugs. Das Verfahren umfasst ein Senden eines Signals, umfassend Authentisierungsinformationen und Zustandsinformationen, von dem medizinischen Implantat an eine Kontrollvorrichtung des Kraftfahrzeugs. Das Verfahren umfasst zudem ein Empfangen des Signals. Außerdem umfasst das Verfahren ein Feststellen einer Gültigkeit oder Ungültigkeit des empfangenen Signals basierend auf den Authentisierungsinformationen. Weiterhin umfasst das Verfahren ein Bereitstellen eines Steuerbefehls, im Falle einer festgestellten Gültigkeit, in Reaktion auf Zustandsinformationen, die einen kritischen Gesundheitszustand des Benutzers anzeigen, an die funktionale Komponente des Kraftfahrzeugs. Die Authentisierungsinformationen erlauben eine zeitlich eindeutige Rückverfolgung eines Ursprungs des Signals.

Somit kann eine Zeit zwischen einem Eintreten einer Zustandsverschlechterung des Benutzers, die für diesen in einigen Fällen nicht unmittelbar wahrnehmbar sein kann, und einer Detektion dieses Zustandes durch das Fahrzeug erheblich verkürzt werden. Ggf. kann das Fahrzeug sogar unmittelbar bei Eintreten des verschlechterten Gesundheitszustandes des Benutzers Maßnahmen ergreifen, um den Benutzer zu warnen oder Verkehrsbehinderungen zu vermeiden. Weiterhin kann es für die Kontrollvorrichtung möglich sein, zwischen einem authentischen Signal und einem falschen Signal zu unterscheiden, um somit z. B. Replay-Angriffen oder Angriffen durch manipulierte Signale vorzubeugen.

Bei einigen Ausführungsbeispielen umfasst das Verfahren ferner ein Ermitteln eines Zeitfensters für eine Gültigkeit des Signals basierend auf den Authentisierungsinformationen. Möglichkeiten für missbräuchliche Anwendungen können so erheblich eingeschränkt werden. Bei manchen Ausführungsbeispielen umfasst das Verfahren ferner ein Ermitteln eines Erzeugungszeitpunktes des Signals aus den Authentisierungsinformationen. Dabei umfasst das Ermitteln des Zeitfensters ein Festlegen eines Endes des Zeitfensters auf einen Zeitpunkt spätestens eine Minute nach dem Erzeugungszeitpunkt des Signals. Dadurch kann eine Aktualität des Signals überprüft werden, und die Authentizität des Signals festgestellt werden. Bei einigen Ausführungsbeispielen umfasst das Verfahren ferner ein Ermitteln einer eindeutigen Zeichenabfolge aus den Authentisierungsinformationen. Dabei umfasst das Ermitteln des Zeitfensters ein Festlegen eines Endes des Zeitfensters unmittelbar nach einem Zeitpunkt, zu dem die eindeutige Zeichenabfolge erstmalig von der Kontrollvorrichtung empfangen wurde. Es kann auf diese Weise möglich werden, ein kryptographisches Geheimnis lediglich dem medizinischen Implantat und der Kontrollvorrichtung zugänglich zu machen, anhand dessen die Authentizität des Signals festgestellt werden kann.

Bei manchen Ausführungsbeispielen umfasst das Verfahren ferner ein Erzeugen der eindeutigen Zeichenabfolge durch die Kontrollvorrichtung. Das Verfahren umfasst außerdem ein Senden der eindeutigen Zeichenabfolge von der Kontrollvorrichtung an das medizinische Implantat. Das Verfahren umfasst weiterhin ein Empfangen der eindeutigen Zeichenabfolge. Dies kann mit anderen Worten ein Generieren des kryptographischen Geheimnisses auf Seiten der Kontrollvorrichtung bedeuten, wodurch eine Wahrscheinlichkeit für einen erfolgreichen Abhörangriff weiter verringert werden kann.

Bei einigen Ausführungsbeispielen umfasst das Verfahren ferner ein Ermitteln einer Passagierposition oder eine Fahrerposition innerhalb des Kraftfahrzeugs, an welcher sich der Benutzer befindet, basierend auf dem Signal. So kann es möglich sein, je nachdem, welcher Benutzer des Fahrzeugs betroffen ist, mit unterschiedlichen Maßnahmen zu reagieren.

Bei manchen Ausführungsbeispielen umfasst das Verfahren ferner ein Senden eines Informationssignals mit Informationen über einen Fahrzeugzustand an das medizinische Implantat. Dies kann ggf. eine spätere Diagnose für medizinisches Hilfspersonal erleichtern, da die Informationen über den Fahrzeugzustand unter Umständen genaueren Aufschluss über eine Ursache der Zustandsverschlechterung des Benutzers erlauben.

Bei einigen Ausführungsbeispielen umfasst das Verfahren ferner ein Abspeichern des Informationssignals. Somit kann die Information nicht nur bis zu einer Diagnose durch medizinisches Personal, sondern auch danach verfügbar sein, beispielsweise für statistische Zwecke oder zur Information des Benutzers, um künftigen Verschlechterungen des Gesundheitszustandes vorzubeugen.

Bei manchen Ausführungsbeispielen umfasst das Verfahren ferner ein Erzeugen des Signals basierend auf einem kritischen Gesundheitszustand eines das medizinische Implantat tragenden Benutzers. Dabei umfasst das Signal die Authentisierungsinformationen und die Zustandsinformationen. Hierdurch kann eine Warnung, ggf. unmittelbar bei Eintreten der Verschlechterung des Zustands des Benutzers, an die Kontrollvorrichtung bereitgestellt werden, und dabei zwischen einer Echtheit der Warnung und einem möglichen unbefugten oder ungewollten Zugriff unterschieden werden.

Ein nicht beanspruchter Aspekt betrifft ein Programm oder Computerprogramm mit einem Programmcode zum Durchführen eines der genannten Verfahren, wenn der Programmcode auf einem Computer, einem Prozessor oder einer programmierbaren Hardwarekomponente, wie z.B. einer applikationsspezifischen integrierten Schaltung (ASIC), ausgeführt wird.

Gemäß einem weiteren Aspekt bezieht sich die Erfindung auf eine Kontrollvorrichtung für ein Kraftfahrzeug. Die Kontrollvorrichtung umfasst einen Empfänger, welcher dazu ausgebildet ist, ein Signal von einem medizinischen Implantat eines Benutzers des Kraftfahrzeugs zu empfangen. Dabei umfasst das Signal Authentisierungsinformationen und Zustandsinformationen. Die Kontrollvorrichtung umfasst zudem ein Überprüfungsmodul, welches dazu ausgebildet ist, basierend auf den Authentisierungsinformationen eine Gültigkeit oder Ungültigkeit des empfangenen Signals festzustellen. Die Kontrollvorrichtung umfasst weiterhin eine Steuereinrichtung, welche im Falle einer festgestellten Gültigkeit dazu ausgebildet ist, in Reaktion auf Zustandsinformationen, die einen kritischen Gesundheitszustand des Benutzers anzeigen, einen Steuerbefehl an eine funktionale Komponente des Kraftfahrzeugs bereitzustellen. Die Authentisierungsinformationen erlauben eine zeitlich eindeutige Rückverfolgung eines Ursprungs des Signals.

Somit kann eine Zeit zwischen einem Eintreten einer Zustandsverschlechterung des Benutzers und einer Detektion dieses Zustandes durch das Fahrzeug erheblich verkürzt werden. Ggf. kann das Fahrzeug sogar unmittelbar bei Eintreten des verschlechterten Gesundheitszustandes des Benutzers Maßnahmen ergreifen, um den Benutzer zu warnen oder Verkehrsbehinderungen zu vermeiden. Weiterhin kann es für die Kontrollvorrichtung möglich sein, zwischen einem authentischen Signal und einem falschen Signal zu unterscheiden, um somit z. B. Replay-Angriffen oder Angriffen durch manipulierte Signale vorzubeugen.

Bei einigen Ausführungsbeispielen ist das Überprüfungsmodul dazu ausgebildet, basierend auf den Authentisierungsinformationen ein Zeitfenster zu ermitteln, und eine Gültigkeit des empfangenen Signals festzustellen, wenn ein Empfangszeitpunkt des Signals innerhalb des Zeitfensters liegt, oder eine Ungültigkeit des empfangenen Signals festzustellen, wenn der Empfangszeitpunkt des Signals außerhalb des Zeitfensters liegt. Möglichkeiten für missbräuchliche Anwendungen können so erheblich eingeschränkt werden.

Bei manchen Ausführungsbeispielen ist das Überprüfungsmodul dazu ausgebildet, aus den Authentisierungsinformationen einen Erzeugungszeitpunkt zu ermitteln, zu dem das Signal erzeugt wurde, und ein Ende des Zeitfensters auf einen Zeitpunkt spätestens eine Minute nach dem Erzeugungszeitpunkt festzulegen. Dadurch kann eine Aktualität des Signals überprüft werden, und darauf basierend die Authentizität des Signals festgestellt werden.

Bei einigen Ausführungsbeispielen ist das Überprüfungsmodul dazu ausgebildet, aus den Authentisierungsinformationen eine eindeutige Zeichenabfolge zu ermitteln, und ein Ende des Zeitfensters unmittelbar nach einem Zeitpunkt festzulegen, zu dem die eindeutige Zeichenabfolge erstmalig von dem Empfänger empfangen wurde. Es kann auf diese Weise möglich werden, ein kryptographisches Geheimnis lediglich dem medizinischen Implantat und der Kontrollvorrichtung zugänglich zu machen, anhand dessen die Authentizität des Signals festgestellt werden kann.

Bei manchen Ausführungsbeispielen ist das Überprüfungsmodul dazu ausgebildet, die eindeutige Zeichenabfolge zu erzeugen. Die Kontrollvorrichtung umfasst ferner einen Sender, welcher dazu ausgebildet ist, die eindeutige Zeichenabfolge an das medizinische Implantat zu senden. Dies kann mit anderen Worten ein Generieren des kryptographischen Geheimnisses auf Seiten der Kontrollvorrichtung bedeuten, wodurch eine Wahrscheinlichkeit für einen erfolgreichen Abhörangriff weiter verringert werden kann.

Bei einigen Ausführungsbeispielen ist das Überprüfungsmodul dazu ausgebildet, basierend auf dem Signal eine Passagierposition oder eine Fahrerposition innerhalb des Kraftfahrzeugs zu ermitteln, an welcher sich der Benutzer befindet. So kann es möglich sein, je nachdem, welcher Benutzer des Fahrzeugs betroffen ist, mit unterschiedlichen Maßnahmen zu reagieren.

Bei manchen Ausführungsbeispielen ist das Überprüfungsmodul dazu ausgebildet, aus den Authentisierungsinformationen eine eindeutig einer Passagierposition oder einer Fahrerposition des Kraftfahrzeugs zugeordnete Kennung zu ermitteln. Die Kennung kann beispielsweise bei einem Zusteigen des Benutzers vergeben werden, oder durch einen Annäherungssensor, der beispielsweise eine Annäherung des Implantats an einen Sitzplatz innerhalb des Fahrzeugs registriert. Alternativ kann die Kennung durch eine manuelle Benutzereingabe vergeben werden.

Bei einigen Ausführungsbeispielen ist das Überprüfungsmodul dazu ausgebildet, aus dem Signal eine auf eine Passagierposition oder eine Fahrerposition des Kraftfahrzeugs hinweisende Ursprungsrichtung und/oder Ursprungsentfernung zu ermitteln. Hierzu können beispielsweise bekannte und gängige Methoden wie z. B. Beamforming eingesetzt werden, welche vergleichsweise aufwandsarm implementierbar sein können.

Bei manchen Ausführungsbeispielen ist die Steuereinrichtung dazu ausgebildet, mittels des Steuerbefehls ein Abbremsen eines Motors des Kraftfahrzeugs oder ein Steuern einer Lenkung des Kraftfahrzeugs zu veranlassen, falls das Signal einem medizinischen Implantat eines Benutzers an der Fahrerposition zugeordnet ist. Dies kann für einen gesundheitlich beeinträchtigten Fahrer ein Führen des Fahrzeugs erheblich vereinfachen, und so möglicherweise ein verbessertes Kontrollieren des Fahrzeugs gestatten.

Bei einigen Ausführungsbeispielen ist die Steuereinrichtung dazu ausgebildet, mittels des Steuerbefehls eine optische Signalausgabe einer Anzeigeeinrichtung des Kraftfahrzeugs oder eine Tonausgabe eines Lautsprechers des Kraftfahrzeugs zu veranlassen, falls das Signal einem medizinischen Implantat eines Benutzers an einer Passagierposition zugeordnet ist. Somit kann der Benutzer rechtzeitig vor einer beginnenden Beeinträchtigung seines Gesundheitszustands gewarnt werden, aber dabei auch ggf. unnötige automatische Eingriffe in eine Führung des Fahrzeugs vermieden werden.

Bei manchen Ausführungsbeispielen umfasst die Kontrollvorrichtung ferner einen Sender, welcher dazu ausgebildet ist, ein Informationssignal mit Informationen über einen Fahrzeugzustand an das medizinische Implantat zu senden. Dies kann ggf. eine spätere Diagnose für medizinisches Hilfspersonal erleichtern, da die Informationen über den Fahrzeugzustand unter Umständen genaueren Aufschluss über eine Ursache der Zustandsverschlechterung des Benutzers erlauben.

Bei einigen Ausführungsbeispielen ist der Sender dazu ausgebildet, das Informationssignal mit Informationen an das medizinische Implantat zu senden, welche eine Fahrzeuginnentemperatur, eine zuletzt am Stück zurückgelegte Kilometerzahl und/oder eine zuletzt am Stück zurückgelegte Fahrzeit umfassen. Derartige Parameter können hilfreich für eine Diagnose sein, da sie ggf. eine Rolle bei einer Zustandsverschlechterung des Benutzers spielen können.

Manche Ausführungsbeispiele beziehen sich zudem auf ein Kraftfahrzeug, welches eine genannte Kontrollvorrichtung umfasst. Dadurch kann implantattragenden Benutzern eine Teilnahme am Straßenverkehr erheblich erleichtert werden.

Gemäß noch einem weiteren Aspekt bezieht sich die Erfindung auf ein medizinisches Implantat. Das medizinische Implantat umfasst einen Signalerzeuger, welcher dazu ausgebildet ist, basierend auf einem kritischen Gesundheitszustand eines das medizinische Implantat tragenden Benutzers ein Signal zu erzeugen. Das Signal umfasst dabei

Authentisierungsinformationen und Zustandsinformationen. Die Authentisierungsinformationen erlauben eine zeitlich eindeutige Rückverfolgung eines Ursprungs des Signals.

Das medizinische Implantat umfasst ferner einen Sender, welcher dazu ausgebildet ist, das Signal an eine Kontrollvorrichtung eines Kraftfahrzeugs zu senden. Dies ermöglicht unter Umständen eine Kommunikation mit dem Fahrzeug, sodass dieses schneller in Kenntnis über eine Verschlechterung des Gesundheitszustands des Benutzers gelangen kann. Eventuelle unterstützende Maßnahmen können so ggf. schneller oder auch automatisch durch die Kontrollvorrichtung ergriffen werden Bei manchen Ausführungsbeispielen ist der Signalerzeuger dazu ausgebildet, das Signal mit den Authentisierungsinformationen zu erzeugen. Dabei umfassen die Authentisierungsinformationen einen Erzeugungszeitpunkt des Signals und/oder eine eindeutige Zeichenabfolge. Das Implantat kann somit von der Kontrollvorrichtung des Fahrzeugs als Quelle des Signals identifiziert werden, wodurch Missbrauch oder Reaktionen auf falsche Alarme vermieden werden können.

Bei einigen Ausführungsbeispielen umfasst das medizinische Implantat ferner einen

Empfänger. Der Empfänger ist dazu ausgebildet, die eindeutige Zeichenabfolge von der Kontrollvorrichtung zu empfangen. Somit kann eine Vereinbarung eines kryptographischen Geheimnisses zwischen dem Implantat und der Kontrollvorrichtung erfolgen.

Bei manchen Ausführungsbeispielen umfasst das medizinische Implantat ferner einen Empfänger, welcher dazu ausgebildet ist, ein Informationssignal mit Informationen über einen Fahrzeugzustand von der Kontrollvorrichtung zu empfangen. Dadurch kann es ermöglicht werden, implantatsseitig zusätzliche Information zu hinterlegen, die bei einer Diagnose hilfreich sein kann.

Bei einigen Ausführungsbeispielen umfasst das medizinische Implantat ferner ein Speichermodul, welches dazu ausgebildet ist, die eindeutige Zeichenabfolge oder das Informationssignal zu speichern. Dies kann ggf. eine Verbesserung einer Diagnose erlauben.

Bei manchen Ausführungsbeispielen ist der Signalerzeuger dazu ausgebildet, ein eindeutiges Identifikationssignal zu erzeugen. Der Sender kann ferner dazu ausgebildet sein, das

Identifikationssignal an eine funktionale Komponente des Kraftfahrzeugs zu senden. Das Implantat kann somit möglicherweise als Zugangsberechtigung verwendet werden, beispielsweise um das Fahrzeug auf- und zuzusperren, oder um einen Motor des Fahrzeugs anzulassen.

Nachfolgend werden unter Bezugnahme auf die beigefügten Figuren einige exemplarische Ausführungsbeispiele näher beschrieben und erläutert. Es zeigen im Einzelnen:
- Fig. 1: ein Ablaufdiagramm eines Verfahrens zur Steuerung einer funktionalen Komponente eines Kraftfahrzeugs mittels eines medizinischen Implantats eines Benutzers des Kraftfahrzeugs gemäß einem Ausführungsbeispiel;
- Fig. 2: ein schematisches Diagramm eines Systems aus medizinischem Implantat und Kontrollvorrichtung eines Kraftfahrzeug und dessen Funktionsweise gemäß einem einfachen Ausführungsbeispiel;
- Fig. 3a: ein Blockdiagramm einer Kontrollvorrichtung eines Kraftfahrzeug gemäß einem detaillierten Ausführungsbeispiel; und
- Fig. 3b: ein Blockdiagramm eines medizinischem Implantats gemäß einem detaillierten Ausführungsbeispiel.

Verschiedene Ausführungsbeispiele werden nun ausführlicher unter Bezugnahme auf die beiliegenden Zeichnungen beschrieben, in denen einige Ausführungsbeispiele dargestellt sind. In den Figuren können die Dickenabmessungen von Linien, Schichten und/oder Regionen um der Deutlichkeit Willen übertrieben dargestellt sein.

Bei der nachfolgenden Beschreibung der beigefügten Figuren, die Ausführungsbeispiele zeigen, bezeichnen gleiche Bezugszeichen gleiche oder vergleichbare Komponenten. Ferner werden zusammenfassende Bezugszeichen für Komponenten und Objekte verwendet, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt.

Obwohl Ausführungsbeispiele auf verschiedene Weise modifiziert und abgeändert werden können, sind Ausführungsbeispiele in den Figuren als Beispiele dargestellt und werden hierin ausführlich beschrieben. Es sei jedoch klargestellt, dass nicht beabsichtigt ist, Ausführungsbeispiele auf die jeweils offenbarten Formen zu beschränken, sondern dass Ausführungsbeispiele vielmehr sämtliche funktionale und/oder strukturelle Modifikationen, Äquivalente und Alternativen, die im Bereich der Erfindung liegen, abdecken sollen. Gleiche Bezugszeichen bezeichnen in der gesamten Figurenbeschreibung gleiche oder ähnliche Elemente.

Man beachte, dass ein Element, das als mit einem anderen Element "verbunden" oder "gekoppelt" bezeichnet wird, mit dem anderen Element direkt verbunden oder gekoppelt sein kann oder dass dazwischenliegende Elemente vorhanden sein können. Wenn ein Element dagegen als "direkt verbunden" oder "direkt gekoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.).

Die Terminologie, die hierin verwendet wird, dient nur der Beschreibung bestimmter Ausführungsbeispiele und soll die Ausführungsbeispiele nicht beschränken. Wie hierin verwendet, sollen die Singularformen " einer", " eine", "eines" und "der", "die", "das" auch die Pluralformen beinhalten, solange der Kontext nicht eindeutig etwas anderes angibt. Ferner sei klargestellt, dass die Ausdrücke wie z.B. "beinhaltet", "beinhaltend", "aufweist" und/oder "aufweisend", "umfasst" und/oder "umfassend" wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

Patienten mit Implantaten, beispielsweise Kardioverter-Defibrillatoren oder Herzschrittmachern, haben einen präzisen Sensor zur Detektion eines medizinischen Notfalls im eigenen Körper implantiert. Der Sensor kann eine präzisere Detektion erlauben als z. B. herkömmliche Fahrerassistenzsysteme, die lediglich Unterschiede in einem Fahrverhalten des Patienten, bzw. Benutzers des Fahrzeugs erfassen. Durch nachfolgende Ausführungsbeispiele kann die Entscheidung des Fahrzeuges, ob sich der Benutzer in einem medizinischen Notfall befindet, zusätzlich auf Basis der Sensordaten möglicher Implantate des Fahrers getroffen werden. Ein Implantat gemäß manchen Ausführungsbeispielen kann z. B. per Funk ausgelesen werden. Sendet das Implantat im Falle der Detektion von z. B. Kammerflimmern dem Fahrzeug diese Information integer und authentisch zu, kann eine Kontrolleinrichtung des Fahrzeugs auf Basis dieser vertrauenswürdigen Information das Fahrzeug schneller und kontrolliert zum Stillstand bringen. Die Sensorik eines Defibrillators kann den medizinischen Notfall des Benutzers unter Umständen sowohl schneller, als auch präziser detektieren. Im Vergleich zu konventionellen Fahrerassistenzsystemen würde z. B. ein Abweichen von der Fahrbahn zeitlich nach dem Kammerflimmern auftreten, und könnte so lediglich zeitverzögert registriert werden. Zudem kann eine Fehlerwahrscheinlichkeit bei Entscheidung über den medizinischen Notfall des Benutzers beim Implantat geringer sein als bei einem Spurhalteassistenten, der womöglich nicht zwischen einem beabsichtigten und notgedrungenen Überfahren einer durchgezogenen Linie unterscheiden kann. Außerdem kann es durch die vorliegenden Ausführungsbeispiele ermöglicht werden, Patienten mit derartigen Implantaten die Angst vor dem Autofahren nehmen.

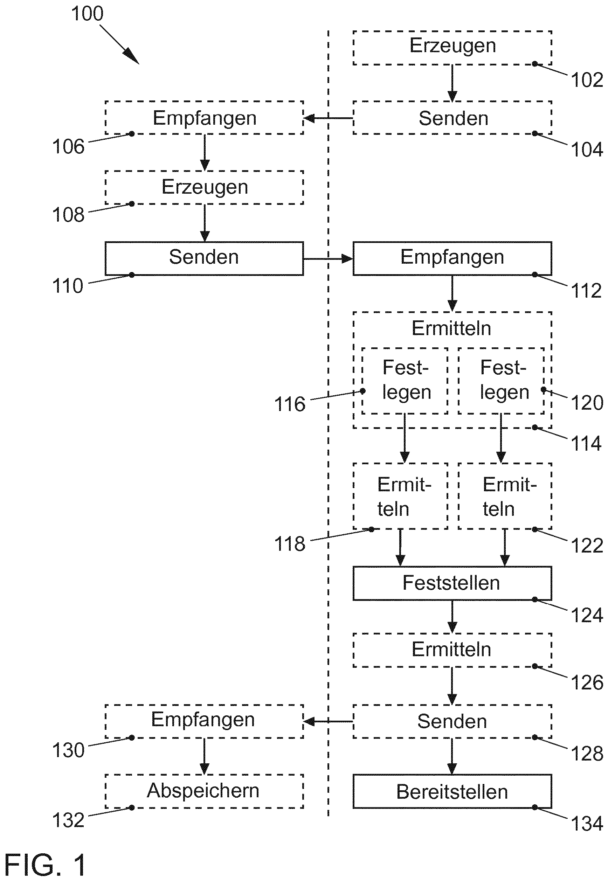

Fig. 1 zeigt ein Ablaufdiagramm eines Verfahrens 100 zur Steuerung einer funktionalen Komponente eines Kraftfahrzeugs mittels eines medizinischen Implantats eines Benutzers des Kraftfahrzeugs. Darin sind solche Verfahrensschritte, die implantatsseitig erfolgen, auf der linken Seite einer gestrichelten Linie, und solche Schritte, die fahrzeugseitig, bzw. durch eine Kontrollvorrichtung des Fahrzeugs erfolgen, auf der rechten Seite der gestrichelten Linie aufgeführt. Zunächst werden Verfahrensschritte eines einfachen Ausführungsbeispiels erläutert, die als durchgezogene Boxen dargestellt sind. Optionale weitere Verfahrensschritte, von denen wahlweise alle, einige oder auch keiner zusätzlich zu den zuvor genannten erfolgen können, sind als gestrichelte Boxen dargestellt, und werden im weiteren Verlauf noch näher beschrieben.

Das Verfahren 100 umfasst ein Senden 110 eines Signals, umfassend Authentisierungsinformationen und Zustandsinformationen, von dem medizinischen Implantat an die Kontrollvorrichtung des Kraftfahrzeugs. Signale können analog oder digital, kontinuierlich oder diskret, periodisch oder aperiodisch sein. Authentisierungsinformationen können solche Informationen sein, die eine eindeutige Rückverfolgung eines Ursprungs des Signals, zeitlich und beispielsweise auch räumlich und bezogen auf einen Absender, erlauben. Dies kann beispielsweise eine Signatur sein. Zustandsinformationen können einen Aufschluss über einen Gesundheitszustand des implantattragenden Benutzers geben. Bei einem einfachen Ausführungsbeispiel kann dies beispielsweise digital erfolgen; so kann z. B. der Wert "low" oder "0" einen unkritischen, und der Wert "high" oder "1" einen kritischen Gesundheitszustand des Benutzers bedeuten. Die Kontrollvorrichtung kann z. B. ein Bordcomputer eines Fahrzeugs oder ein Fahrerassistenzsystem sein. Ein medizinisches Implantat kann z. B. ein Herzschrittmacher, Kardioverter-Defibrillator, eine Insulinpumpe, ein Gerät für eine Tiefe Hirnstimulation, etc. sein.

Das Verfahren 100 umfasst zudem ein Empfangen 112 des Signals. Das Empfangen 112 kann durch einen kontrollvorrichtungsseitigen Empfänger erfolgen. Außerdem umfasst das Verfahren 100 ein Feststellen 124 einer Gültigkeit oder Ungültigkeit des empfangenen Signals durch die Kontrollvorrichtung. Weiterhin umfasst das Verfahren 100 ein Bereitstellen 134 eines Steuerbefehls von der Kontrollvorrichtung, im Falle einer festgestellten Gültigkeit, in Reaktion auf Zustandsinformationen, die einen kritischen Gesundheitszustand des Benutzers anzeigen, an die funktionale Komponente des Kraftfahrzeugs. Funktionale Komponenten können z. B. eine Lenkung, einen Motor, eine Anzeige, eine Kontrollleuchte, eine Hupe oder einen Lautsprecher umfassen. Eine Zustandsverschlechterung des Benutzers kann für diesen in einigen Fällen nicht unmittelbar wahrnehmbar sein, und so durch das Fahrzeug wenigstens ein Hinweis erfolgen, oder sogar fahrerunterstützende Maßnahmen ergriffen werden. Dies kann ein durch die Kontrollvorrichtung vorgenommenes bzw. computergestütztes Führen des Fahrzeugs umfassen. Weiterhin kann z. B. eine Ungültigkeit des Signals auf einen Wiedergabe- (Replay-) Angriff oder falschen Alarm hindeuten. Replay-Angriffe können allgemein dadurch erfolgen, dass ein Signal von einem weiteren Gerät abgehört, kopiert, und zu einem späteren Zeitpunkt, den ein unbefugter Angreifer frei wählen kann, wiedergegeben wird. Hierdurch kann z. B. ein gezieltes Abstoppen von Fahrzeugen durch einen unbefugten Angreifer womöglich verhindert werden. Weiterhin könnten falsche Alarme in herkömmlichen Fällen möglicherweise durch routinemäßige Signale ausgelöst werden, die unter Umständen von dem Implantat an die Kontrollvorrichtung versendet werden, jedoch keine Zustandsinformation über einen kritischen Gesundheitszustand des Benutzers umfassen. Daher kann bei einem Ausführungsbeispiel eine Authentisierungsinformation bei einem solchen routinemäßigen Signal entfallen.

Bei einigen Ausführungsbeispielen umfasst das Verfahren 100 ferner ein Ermitteln 114 eines Zeitfensters für eine Gültigkeit des Signals basierend auf den Authentisierungsinformationen. Das Zeitfenster kann dabei wenigstens nach hinten begrenzt sein. Somit kann ein gültiges Signal z. B. dadurch charakterisiert sein, dass eine Authentisierungsinformation eine Bedingung erfüllt, die auf eine zeitliche Lage des Signals innerhalb des Zeitfensters hinweist. Umgekehrt kann eine zeitliche Lage außerhalb des Zeitfensters eine Ungültigkeit des Signals bedeuten.

Gemäß einer Variante umfasst das Verfahren 100 ein Ermitteln 118 eines Erzeugungszeitpunktes des Signals aus den Authentisierungsinformationen. Der Erzeugungszeitpunkt kann z. B. ein Datum und eine Uhrzeit, insbesondere eine wenigstens minuten-, bevorzugt aber sekundengenaue Uhrzeit umfassen. Dabei umfasst das Ermitteln 114 des Zeitfensters ein Festlegen 116 eines Endes des Zeitfensters auf einen Zeitpunkt spätestens eine Minute nach dem Erzeugungszeitpunkt des Signals. Der Zeitpunkt kann alternativ auch spätestens fünf Minuten, zehn Sekunden oder eine Sekunde nach dem Erzeugungszeitpunkt liegen. Ein Beginn des Zeitfensters kann ferner beispielsweise auf den Erzeugungszeitpunkt festgelegt werden. Dadurch kann mittels Überprüfung der Authentizität des Signals oder der Integrität einer Nonce die Aktualität oder Gültigkeit des Signals festgestellt werden.

Gemäß einer anderen Variante umfasst das Verfahren 100 ferner ein Ermitteln 122 einer eindeutigen Zeichenabfolge aus den Authentisierungsinformationen. Dabei umfasst das Ermitteln 114 des Zeitfensters ein Festlegen 120 eines Endes des Zeitfensters unmittelbar nach einem Zeitpunkt, zu dem die eindeutige Zeichenabfolge erstmalig von der Kontrollvorrichtung empfangen wurde. Dabei kann sich ein derartiges Empfangen auf ein Empfangen von einer Quelle außerhalb der Kontrollvorrichtung beziehen. Im Gegensatz dazu kann z. B. ein Sendeempfänger der Kontrollvorrichtung die eindeutige Zeichenabfolge von einem die Zeichenabfolge generierenden Modul der Kontrollvorrichtung empfangen, was hierbei jedoch unberücksichtigt bleiben kann. Der Sendeempfänger kann anschließend die Zeichenabfolge an das medizinische Implantat senden. Ein Beginn des Zeitfensters kann ferner beispielsweise auf einen Zeitpunkt, zu dem die eindeutige Zeichenabfolge erstmalig von dem medizinischen Implantat empfangen wurde, festgelegt werden.

Die Zeichenabfolge kann beispielsweise ein Zähler oder eine laufende Zahl sein. Die Zeichenabfolge kann weiterhin eine bestimmte Nonce oder Transaktionsnummer sein, welche zufallsbasiert und/oder aus einer seitens der Kontrollvorrichtung abgespeicherten Tabelle heraus erzeugt wird. Die Zeichenabfolge kann ihre Gültigkeit nach einmaliger Verwendung verlieren; insbesondere kann die Zeichenabfolge z. B. die Gültigkeit eines Signals ausschließlich für die nächste Transaktion (das Senden 110 und das Empfangen 112) belegen. Die Zeichenabfolge kann eine Funktion der Nonce und eines kryptographischen Geheimnisses darstellen, welches lediglich dem medizinischen Implantat und der Kontrollvorrichtung zugänglich ist. Die Gültigkeit des Signals kann durch die Zeichenabfolge belegt werden. Das Zeitfenster kann ferner zu einem Zeitpunkt beginnen, zu dem das Implantat in Kenntnis der Zeichenabfolge gelangt.

Dabei kann das Verfahren 100 ein Erzeugen 102 der eindeutigen Zeichenabfolge durch die Kontrollvorrichtung umfassen. Das Erzeugen 102 kann, wie beschrieben, mittels eines Zufallsgenerators, durch Auswählen aus einer vorgefertigten Tabelle, oder anderweitig erfolgen. Die Zeichenabfolge kann bei einem einfachen Ausführungsbeispiel einen Binärcode, oder numerische, alphanumerische und/oder Sonderzeichen umfassen. In einem weiteren Sinne kann die Zeichenabfolge auch ein eindeutiger Frequenz- oder Zeitverlauf eines elektromagnetischen Kommunikationssignals sein. Das Verfahren 100 umfasst bei einem solchen Ausführungsbeispiel außerdem ein Senden 104 der eindeutigen Zeichenabfolge von der Kontrollvorrichtung an das medizinische Implantat. Das Verfahren 100 umfasst weiterhin ein Empfangen 106 der eindeutigen Zeichenabfolge.

Bei manchen Ausführungsbeispielen umfasst das Verfahren 100 ferner ein Erzeugen 108 des Signals basierend auf einem kritischen Gesundheitszustand eines das medizinische Implantat tragenden Benutzers. Dabei umfasst das Signal die Authentisierungsinformationen und die Zustandsinformationen. Das Senden 110 des Signals, oder mit anderen Worten, einer Warnung an die Kontrollvorrichtung über eine Verschlechterung des Gesundheitszustands des Benutzers, kann so insbesondere unmittelbar bei einem Eintreten der Zustandsverschlechterung erfolgen. Dabei kann zwischen einer Echtheit der Warnung und einem möglichen unbefugten oder unbeabsichtigten Zugriff unterschieden werden.

Bei einigen Ausführungsbeispielen umfasst das Verfahren 100 ferner ein Ermitteln 126 einer Benutzerposition innerhalb des Kraftfahrzeugs, basierend auf dem Signal. Die Benutzerposition kann eine Passagierposition auf einem Beifahrer- oder Rücksitz, oder eine Fahrerposition auf einem Fahrersitz sein, an welcher sich der Benutzer befindet. Beispielsweise kann das Ermitteln 126 der Benutzerposition mittels Beamforming durchgeführt werden. Eine Ursprungsposition des Signals kann z. B. aus Laufzeitunterschieden des Signals zu mehreren verschiedenen Empfängern der Kontrollvorrichtung ermittelt werden. Ferner kann auch z. B. aus dem Signal eine Information gewonnen werden, die auf eine Position bezogen ist. So kann sich z. B. ein implantattragender Benutzer durch ein manuelles Eingeben der Kontrollvorrichtung als der Fahrer des Fahrzeugs identifizieren, und alternativ oder zusätzlich ein weiterer implantattragender Benutzer als Passagier. Dadurch kann es möglich sein, je nachdem, welcher Benutzer des Fahrzeugs betroffen ist, mit unterschiedlichen Maßnahmen zu reagieren.

Bei manchen Ausführungsbeispielen umfasst das Verfahren 100 ferner ein Senden 128 eines Informationssignals mit Informationen über einen Fahrzeugzustand an das medizinische Implantat. Informationen über den Fahrzeugzustand können z. B. eine Innentemperatur des Fahrzeugs (beispielsweise zum Zeitpunkt der Empfangens 112 des Signals, aber auch über einen bestimmten Zeitraum), eine zuletzt am Stück gefahrene Kilometerzahl oder Streckendauer sein. Derartige Informationen können unter Umständen genaueren Aufschluss über eine Ursache der Zustandsverschlechterung des Benutzers erlauben. Dies kann ggf. hilfreich für medizinisches Hilfspersonal oder den Benutzer selbst sein.

Entsprechend kann das Verfahren 100 ein Empfangen 130 des Informationssignals umfassen. Bei einigen Ausführungsbeispielen umfasst das Verfahren 100 zudem ein Abspeichern 132 des Informationssignals. Somit kann die Information nicht nur bis zu einer Diagnose durch medizinisches Personal, sondern auch danach verfügbar sein, beispielsweise für statistische Zwecke.

Das Verfahren 100 kann für ein System, welches ein medizinisches Implantat und eine Kontrollvorrichtung eines Kraftfahrzeugs umfasst, ausführbar sein. Weiterhin können Teile des Verfahrens 100 für sich genommen weitere Verfahren darstellen, die lediglich von dem medizinischen Implantat oder der Kontrollvorrichtung ausführbar sein können. Gemäß einem Ausführungsbeispiel umfasst ein weiteres Verfahren für die Kontrollvorrichtung des Kraftfahrzeugs zumindest das Erzeugen 102 und das Senden 104 der eindeutigen Zeichenabfolge an das medizinische Implantat. Ein solches Verfahren kann alternativ, ergänzend oder zusätzlich zu anderen Ausführungsbeispielen oder ggf. mit weiteren Verfahrensschritten ausgeführt werden.

Gemäß einem anderen Ausführungsbeispiel umfasst ein weiteres Verfahren für das medizinische Implantat zumindest das Empfangen 106 der eindeutigen Zeichenabfolge, das Erzeugen 108 und das Senden 110 des Signals. Das Erzeugen 108 geschieht dabei basierend auf einem kritischen Gesundheitszustand des implantattragenden Benutzers, und derart, dass das Signal Authentisierungsinformationen umfasst, die wiederum die eindeutige Zeichenabfolge umfassen. Zusätzlich umfasst das Signal Zustandsinformationen über den Gesundheitszustand des Benutzers.

Gemäß noch einem Ausführungsbeispiel umfasst ein weiteres Verfahren für die Kontrollvorrichtung des Kraftfahrzeugs zumindest das Empfangen 112 des Signals, das Feststellen 124 der Gültigkeit des Signals und das Bereitstellen 134 des Steuerbefehls, im Falle einer festgestellten Gültigkeit, an die funktionale Komponente des Kraftfahrzeugs. Alternativ kann nach dem Empfangen 112 ein Feststellen einer Ungültigkeit des Signals erfolgen, und eine Erzeugung eines Steuerbefehls entfallen. Optional kann das Ermitteln 114 des Zeitfensters dem Empfangen 112 des Signals folgen, und dabei das Festlegen 116; 120 des Endes des Zeitfensters nach zuvor beschriebenen Varianten umfassen. Dabei erfolgt, vorher, zeitgleich oder anschließend, das Ermitteln 122 der eindeutigen Zeichenabfolge, oder alternativ das Ermitteln 118 des Erzeugungszeitpunkts des Signals. Darauf basierend erfolgt das Feststellen 124 der Gültigkeit des Signals und das Ermitteln 126 der Position des implantattragenden Benutzers. Optional umfasst das Verfahren das Senden 128 der Informationen über den Fahrzeugzustand an das Implantat.

Gemäß noch einem weiteren Ausführungsbeispiel umfasst ein weiteres Verfahren für das medizinische Implantat zumindest das Empfangen 130 und das Abspeichern 132 der Informationen über den Fahrzeugzustand.

Die vier letztgenannten Ausführungsbeispiele von implantatsseitigen und kontrollvorrichtungsseitigen Verfahren können jeweils für sich genommen, oder in beliebigen Kombinationen zueinander anwendbar sein. Die Verfahren können beispielsweise auf Prozessoren, Mikrocontrollern oder anderen programmierbaren Hardwarekomponenten ausführbar sein und implementiert werden. Hierzu kann eine Implementierung mithilfe eines Computerprogramms umfassend einen Algorithmus zum Durchführen des jeweiligen Verfahrens vorgenommen werden.

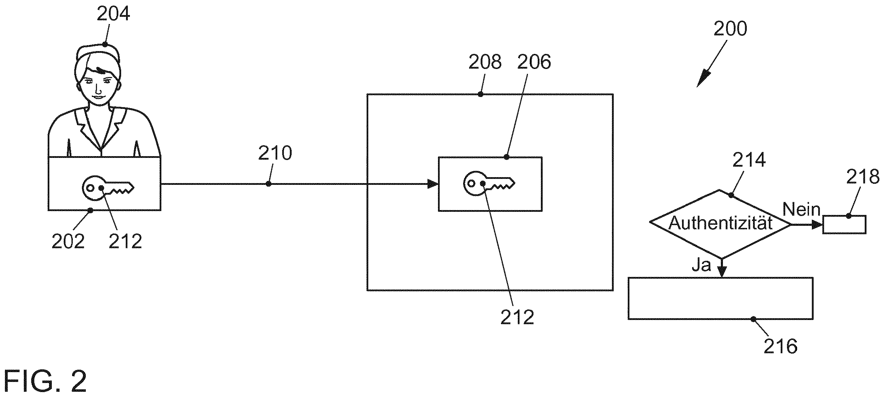

Fig. 2 zeigt ein einfaches Ausführungsbeispiel für ein System 200, welches ein medizinisches Implantat 202 eines Benutzers 204 und eine Kontrollvorrichtung 206 eines Fahrzeugs 208 umfasst. Das Implantat 202 ist beispielsweise ein Defibrillator oder Herzschrittmacher. Die Kontrollvorrichtung 206 ist beispielsweise ein Fahrzeugcomputer oder Fahrerassistenzsystem. Implantat 202 und Kontrollvorrichtung 206 können z. B. per Funkverbindung miteinander in Kontakt stehen. Im medizinischen Notfall oder bei kritisch werdendem Gesundheitszustand des Benutzers 204 kann eine authentische und integre Meldung per Funk an die Kontrollvorrichtung 206 des Fahrzeugs 208 gesendet werden. Die Meldung kann ein Signal 210 sein, welches eine Authentisierungsinformation 212 aufweist. Die Authentisierungsinformation 212 kann ein kryptographischer Schlüssel sein, welcher von der Kontrollvorrichtung 206 zu einer Überprüfung 214 der Authentizität oder Gültigkeit der Meldung herangezogen wird. Bei ermittelter Authentizität folgt ein Ausführen 216 einer Funktion, z. B. eines kontrollierten Anhaltens des Fahrzeugs durch einen hierzu geeigneten Algorithmus. Bei fehlender Authentizität folgt ein Abbruch 218 des Algorithmus.

Das Fahrzeug 208 kann sich anders ausgedrückt ein kryptographisches Geheimnis mit dem Implantat 202 des Fahrers 204 teilen. Detektiert das Implantat 202 eine medizinische Notsituation, kann es eine authentische und integre Information 210 darüber per Funk senden. Das Fahrzeug 208 kann während der Fahrt auf derartige Funkbotschaften hören. Wird eine solche Nachricht erkannt, kann dessen Authentizität verifiziert, und das Fahrzeug 208 sofort und kontrolliert zum Stillstand gebracht werden.

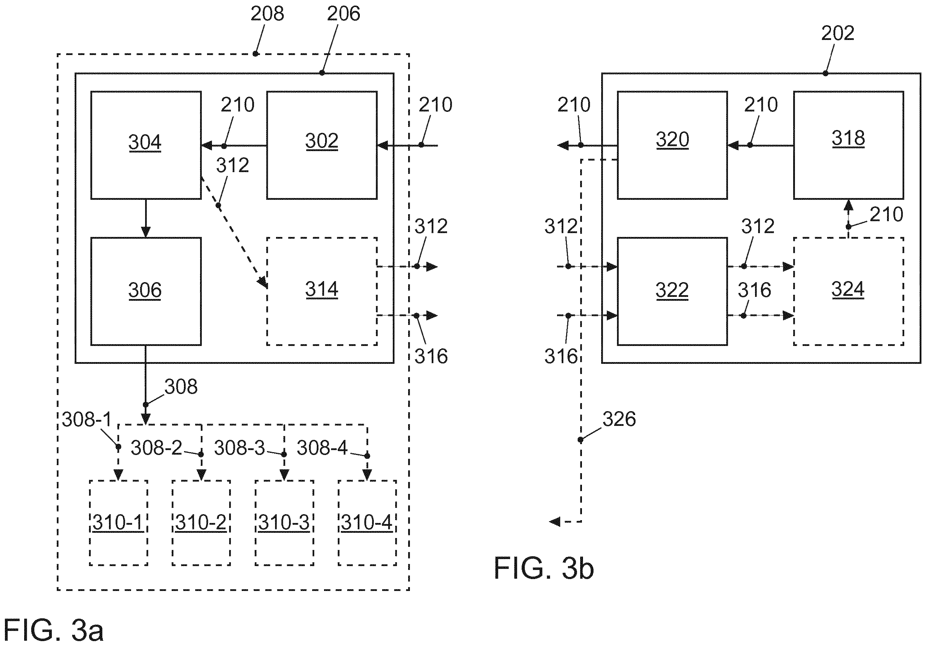

Fig. 3a zeigt ein noch detaillierteres Ausführungsbeispiel einer Kontrollvorrichtung 206 für ein Kraftfahrzeug 208. Kontrollvorrichtung 206 umfasst einen Empfänger 302, welcher dazu ausgebildet ist, ein Signal 210 von einem medizinischen Implantat eines Benutzers des Kraftfahrzeugs 208 zu empfangen. Empfänger 302 kann beispielsweise an einen Sender gekoppelt, oder mit einem Sender gemeinsam als Sendeempfänger ausgeführt sein. Beispielsweise kann Empfänger 302 somit eine Antenne oder ein Array aus mehreren Antennen umfassen. Das Signal 210 umfasst Authentisierungsinformationen und Zustandsinformationen. Kontrollvorrichtung 206 umfasst zudem ein Überprüfungsmodul 304, welches dazu ausgebildet ist, basierend auf den Authentisierungsinformationen eine Gültigkeit oder Ungültigkeit des empfangenen Signals 210 festzustellen. Überprüfungsmodul 304 kann z. B. als Prozessor, Mikrocontroller oder andere programmierbare Hardwarekomponente ausgeführt sein. Überprüfungsmodul 304 kann ferner einen Speicher zum Abspeichern von Daten, z. B. Tabellen, Algorithmen oder kryptographischer Information umfassen. Kontrollvorrichtung 206 umfasst weiterhin eine Steuereinrichtung 306, welche im Falle einer festgestellten Gültigkeit dazu ausgebildet ist, in Reaktion auf Zustandsinformationen, die einen kritischen Gesundheitszustand des Benutzers anzeigen, einen Steuerbefehl 308 an eine funktionale Komponente 310 des Kraftfahrzeugs 208 bereitzustellen. Steuereinrichtung 306 kann entsprechend dem Überprüfungsmodul 304 ausgeführt sein, oder mit diesem sogar als gemeinsame Hardwarekomponente ausgeführt sein. So kann beispielsweise ein Prozessor Funktionalitäten des Überprüfungsmoduls 304 und der Steuereinrichtung 306 übernehmen. Es kann für die Kontrollvorrichtung 206 möglich sein, zwischen einem authentischen Signal 210 und einem falschen Signal zu unterscheiden, und somit z. B. Replay-Angriffen vorzubeugen. Bei Feststellen eines falschen oder ungültigen Signals kann der Steuerbefehl 308 entfallen.

Beispielsweise ist Überprüfungsmodul 304 dazu ausgebildet, basierend auf den Authentisierungsinformationen ein Zeitfenster zu ermitteln, und eine Gültigkeit des empfangenen Signals 210 festzustellen, wenn ein Empfangszeitpunkt des Signals 210 innerhalb des Zeitfensters liegt, oder eine Ungültigkeit des empfangenen Signals festzustellen, wenn der Empfangszeitpunkt des Signals außerhalb des Zeitfensters liegt.

Gemäß einer bereits im Zusammenhang mit Fig. 1 beschriebenen Variante ist Überprüfungsmodul 304 dazu ausgebildet, aus den Authentisierungsinformationen einen Erzeugungszeitpunkt zu ermitteln, zu dem das Signal 210 erzeugt wurde, und ein Ende des Zeitfensters auf einen Zeitpunkt, der beispielsweise spätestens fünf Minuten, eine Minute, zehn Sekunden, eine Sekunde oder lediglich einen Sekundenbruchteil nach dem Erzeugungszeitpunkt liegt, festzulegen. Mittels des Überprüfungsmoduls 304 kann somit eine Aktualität des Signals 210 überprüft werden.

Gemäß einer weiteren im Zusammenhang mit Fig. 1 beschriebenen Variante ist Überprüfungsmodul 304 dazu ausgebildet, aus den Authentisierungsinformationen eine eindeutige Zeichenabfolge 312 zu ermitteln, und ein Ende des Zeitfensters unmittelbar nach einem Zeitpunkt festzulegen, zu dem die eindeutige Zeichenabfolge 312 erstmalig von Empfänger 302 empfangen wurde. Es kann sich bei der eindeutigen Zeichenabfolge 312 um ein kryptographisches Geheimnis handeln, welches lediglich dem medizinischen Implantat und der Kontrollvorrichtung 206 zugänglich ist, und anhand dessen somit die Authentizität des Signals 210 feststellbar ist.

Hierzu kann Überprüfungsmodul 304 dazu ausgebildet sein, die eindeutige Zeichenabfolge 312 zu erzeugen. Kontrollvorrichtung 206 umfasst ferner einen Sender 314, welcher dazu ausgebildet ist, die eindeutige Zeichenabfolge 312 an das medizinische Implantat zu senden. Idealerweise kann dadurch das kryptographische Geheimnis zunächst lediglich der Kontrollvorrichtung 206 bekannt sein, und mit dem Implantat geteilt werden, wodurch eine Wahrscheinlichkeit für einen erfolgreichen Abhörangriff und/oder Replay-Angriff weiter verringert werden kann. Sender 314 kann als gemeinsames Bauteil zusammen mit Empfänger 302, oder mit anderen Worten, als Sendeempfänger ausgeführt sein. Ebenso kann Sender 314 eine Antenne oder ein Array aus mehreren Antennen umfassen.

Bei einigen Ausführungsbeispielen ermittelt Überprüfungsmodul 304 basierend auf Signal 210 eine Passagierposition oder eine Fahrerposition innerhalb des Kraftfahrzeugs, an welcher sich der Benutzer befindet. Beispielsweise kann Überprüfungsmodul 304 dazu ausgebildet sein, aus den Authentisierungsinformationen eine eindeutig einer Passagierposition oder einer Fahrerposition des Kraftfahrzeugs 208 zugeordnete Kennung zu ermitteln. Die Kennung kann beispielsweise bei einem Zusteigen des Benutzers, durch einen Positionssensor zur Lokalisierung des Implantats an einem bestimmten Sitzplatz innerhalb des Fahrzeugs 208, oder durch eine manuelle Benutzereingabe vergeben werden.

Ferner ist Überprüfungsmodul 304 beispielsweise dazu ausgebildet, aus dem Signal 210 eine auf eine Passagierposition oder eine Fahrerposition des Kraftfahrzeugs hinweisende Ursprungsrichtung und/oder Ursprungsentfernung des Signals 210 zu ermitteln. Hierzu können beispielsweise bekannte und gängige Methoden wie z. B. Beamforming eingesetzt werden.

Weiterhin ist Steuereinrichtung 306 beispielsweise dazu ausgebildet, mittels des Steuerbefehls 308 ein Abbremsen 308-1 eines Motors 310-1 des Kraftfahrzeugs 208 oder ein Steuern 308-2 einer Lenkung 310-2 des Kraftfahrzeugs 208 zu veranlassen, falls das Signal 210 einem medizinischen Implantat eines Benutzers an der Fahrerposition zugeordnet ist. Fahrzeug 208 kann so kontrolliert und sicher zum Stillstand gebracht werden. Optional oder alternativ ist Steuereinrichtung 306 dazu ausgebildet, mittels des Steuerbefehls 308 eine optische Signalausgabe 308-3 einer Anzeigeeinrichtung 310-3 des Kraftfahrzeugs 208 (z. B. eines Bildschirms eines Infotainmentsystems oder einer Kontrollleuchte) oder eine Tonausgabe 308-4 eines Lautsprechers 310-4 oder auch einer Hupe des Kraftfahrzeugs 208 zu veranlassen, falls das Signal 210 einem medizinischen Implantat eines Benutzers an einer Passagierposition zugeordnet ist. Es können so einerseits erforderliche oder unterstützende Maßnahmen ergriffen, und andererseits unnötige oder sogar möglicherweise verkehrsbehindernde Maßnahmen vermieden werden. Ferner kann die Zustandsinformation des Signals 210 in einem weiteren Ausführungsbeispiel sogar Aufschluss über einen Schweregrad der Beeinträchtigung des Gesundheitszustandes des Benutzers liefern. Beispielsweise kann mit Hilfe eines oder mehrerer Grenzwerte eine Abstufung erfolgen, ob der Gesundheitszustand lediglich eine Fahrpause des Benutzers oder sogar medizinische Maßnahmen durch ärztliches Fachpersonal erfordert. Entsprechend können die beschriebenen Maßnahmen gewählt werden, z. B. ein Fahrer bei festgestelltem Erschöpfungszustand, der noch keine medizinischen Maßnahmen erforderlich macht, mittels optischer Signalausgabe 308-3 oder Tonausgabe 308-4 zum Anhalten des Fahrzeugs 208 aufgefordert werden.

Optional oder alternativ kann Sender 314 dazu ausgebildet sein, ein Informationssignal 316 mit Informationen über einen Fahrzeugzustand an das medizinische Implantat zu senden. Die Informationen können eine Fahrzeuginnentemperatur, eine zuletzt am Stück zurückgelegte Kilometerzahl und/oder eine zuletzt am Stück zurückgelegte Fahrzeit umfassen. Anders ausgedrückt können außerdem Daten, wie z.B. Sensordaten, in das Implantat übertragen werden. So können im Implantat gespeicherte Daten um Daten der Umgebung, die dem Auto zum gegeben Zeitpunkt bekannt waren, erweitert werden. Beispielsweise könnte bei Eintreten einer medizinischen Notsituation die Fahrzeuginnentemperatur sicher zu dem Implantat übertragen und dort abgespeichert werden. Dies könnte einem Arzt bei einer genaueren Diagnose, oder auch einer Klärung von Versicherungsansprüchen im Falle eines Unfalls dienen.

Manche Ausführungsbeispiele beziehen sich zudem auf das Kraftfahrzeug 208, welches eine genannte Kontrollvorrichtung 206 umfasst. Kraftfahrzeug 208 kann insbesondere ein Landfahrzeug, beispielsweise Personen- oder Lastkraftwagen, aber auch unter Umständen ein Wasser- oder Luftfahrzeug sein.

Es kann durch Ausführungsbeispiele ermöglicht werden, eine sichere Funkverbindung zwischen Fahrzeug 208 und dem medizinischen Implantat herzustellen. Allgemein können Daten, z. B. Signal 210, die eindeutige Zeichenabfolge 312 und die Informationen 316 über den Fahrzeugzustand vor dem Senden verschlüsselt, und nach Empfang wieder entschlüsselt werden. Eine Verschlüsselung kann dabei sowohl fahrzeug- als auch implantatsseitig erfolgen.

Fig. 3b zeigt ein detaillierteres Ausführungsbeispiel eines medizinischen Implantats 202. Das medizinische Implantat 202 umfasst einen Signalerzeuger 318, welcher dazu ausgebildet ist, basierend auf einem kritischen Gesundheitszustand eines das medizinische Implantat 202 tragenden Benutzers das Signal 210 zu erzeugen. Signal 210 umfasst dabei die Authentisierungsinformationen und Zustandsinformationen. Das medizinische Implantat 202 umfasst ferner einen Sender 320, welcher dazu ausgebildet ist, das Signal 210 an die Kontrollvorrichtung 206 des Kraftfahrzeugs 208 (vgl. Fig. 3a) zu senden.

Bei manchen Ausführungsbeispielen, bei denen Signalerzeuger 318 das Signal 210 mit den Authentisierungsinformationen erzeugt, umfassen die Authentisierungsinformationen den Erzeugungszeitpunkt des Signals 210 und/oder die eindeutige Zeichenabfolge 312. Implantat 202 kann somit von Kontrollvorrichtung 206 des Fahrzeugs 208 als Quelle des Signals 210 identifiziert werden, wodurch Missbrauch oder Reaktionen auf falsche Alarme vermieden werden können.

Ferner kann das medizinische Implantat 202 einen Empfänger 322 aufweisen. Sender 320 und Empfänger 322 des medizinischen Implantats 202 können wiederum als gemeinsamer Sendeempfänger ausgebildet sein, und beispielsweise eine oder mehrere Antennen umfassen. Empfänger 322 ist dazu ausgebildet, die eindeutige Zeichenabfolge 312 von Kontrollvorrichtung 206 zu empfangen. Optional kann Implantat 202 dazu ausgebildet sein, eine Bestätigung eines Empfangs der eindeutigen Zeichenabfolge 312 an Kontrollvorrichtung 206 übermitteln.

Ferner kann Empfänger 322 dazu ausgebildet sein, das Informationssignal 316 mit Informationen über den Fahrzeugzustand von Kontrollvorrichtung 206 zu empfangen. Zudem kann das medizinische Implantat ferner ein Speichermodul 324 umfassen, welches dazu ausgebildet ist, die eindeutige Zeichenabfolge 312 oder Informationssignal 316 zu speichern.

Bei manchen Ausführungsbeispielen ist Signalerzeuger 324 dazu ausgebildet, ein eindeutiges Identifikationssignal 326 zu erzeugen. Sender 320 kann ferner dazu ausgebildet sein, das Identifikationssignal 326 an eine funktionale Komponente 310 des Kraftfahrzeugs 208 zu senden. Implantat 202 kann somit möglicherweise als Zugangsberechtigung verwendet werden, beispielsweise um Fahrzeug 208 auf- und zuzusperren, oder um einen Motor 310-1 des Fahrzeugs 208 anzulassen. Ein Zugang zum Fahrzeug 208 kann sogar verwehrt werden, wenn Implantat 202 beispielsweise nach gültiger Authentifizierung zu viel Alkohol im Blut festgestellt hat. Neben dem Ansteuern von Fahrzeugfunktionen können auch Daten zur weiteren Verarbeitung von Implantat 202 in das Fahrzeug 208 übertragen werden.

Ausführungsbeispiele können weiterhin auch in zukünftigen Fahrzeugen, für die ein System implementiert ist, welches dazu ausgebildet ist, das Fahrzeug kontrolliert zum Stillstand zu bringen, eingesetzt werden. Dabei kann das Fahrzeug ein Funkverfahren des Implantats unterstützen. Eine sichere Kommunikation eines Implantats per Funk mit anderen Entitäten kann auf weitere Bereiche über fahrzeugbezogene Anwendungen hinaus übertragen werden. So kann beispielsweise ein Implantat als Zugangsberechtigung dienen. Diese Zugangsberechtigung kann zum einen per Identitätsbeweis mit einem kryptographischen Geheimnis erlangt werden. Darüber hinaus können beliebige weitere Vorbedingungen an den Zugang geknüpft werden. Beispielsweise kann eine Vorbedingung zum Öffnen einer Tresortür sein, dass das Implantat einen Herzschlag feststellt, und sich die Frequenz in einem bestimmten Bereich befindet.

Die in der vorstehenden Beschreibung, den nachfolgenden Ansprüchen und den beigefügten Figuren offenbarten Merkmale können sowohl einzeln wie auch in beliebiger Kombination für die Verwirklichung eines Ausführungsbeispiels in ihren verschiedenen Ausgestaltungen von Bedeutung sein und implementiert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

### Bezugszeichenliste

- 100: Verfahren
- 102: Erzeugen (einer eindeutigen Zeichenabfolge)
- 104: Senden (der eindeutigen Zeichenabfolge)
- 106: Empfangen (der eindeutigen Zeichenabfolge)
- 108: Erzeugen (eines Signals)
- 110: Senden (des Signals)
- 112: Empfangen (des Signals)
- 114: Ermitteln (eines Zeitfensters für eine Gültigkeit des Signals)
- 116: Festlegen (eines Endes des Zeitfensters)
- 118: Ermitteln (eines Erzeugungszeitpunktes des Signals)
- 120: Festlegen (eines Endes des Zeitfensters)
- 122: Ermitteln (einer eindeutigen Zeichenabfolge)
- 124: Feststellen (einer Gültigkeit oder Ungültigkeit des Signals)
- 126: Ermitteln (einer Benutzerposition innerhalb des Kraftfahrzeugs)
- 128: Senden (eines Informationssignals)
- 130: Empfangen (des Informationssignals)
- 132: Abspeichern (des Informationssignals)
- 134: Bereitstellen (eines Steuerbefehls)
- 200: System
- 202: Medizinisches Implantat
- 204: Benutzer
- 206: Kontrollvorrichtung
- 208: Kraftfahrzeug
- 210: Signal
- 212: Authentisierungsinformation
- 214: Überprüfung
- 216: Ausführen
- 218: Abbruch
- 302: Empfänger
- 304: Überprüfungsmodul
- 306: Steuereinrichtung
- 308: Steuerbefehl
- 308-1: Abbremsen
- 308-2: Steuern
- 308-3: Optische Signalausgabe
- 308-4: Tonausgabe
- 310: Funktionale Komponente
- 310-1: Motor
- 310-2: Lenkung
- 310-3: Anzeigeeinrichtung
- 310-4: Lautsprecher
- 312: eindeutige Zeichenabfolge
- 314: Sender
- 316: Informationssignal
- 318: Signalerzeuger
- 320: Sender
- 322: Empfänger
- 324: Speichermodul
- 326: Identifikationssignal

## Patentansprüche

1. Verfahren (100) zur Steuerung einer funktionalen Komponente eines Kraftfahrzeugs mittels eines medizinischen Implantats eines Benutzers des Kraftfahrzeugs, umfassend:
Senden (110) eines Signals, umfassend Authentisierungsinformationen und
Zustandsinformationen, von dem medizinischen Implantat an eine Kontrollvorrichtung des Kraftfahrzeugs;
Empfangen (112) des Signals;
Feststellen (124) einer Gültigkeit oder Ungültigkeit des empfangenen Signals basierend auf den Authentisierungsinformationen;
Bereitstellen (134) eines Steuerbefehls, im Falle einer festgestellten Gültigkeit, in Reaktion auf Zustandsinformationen, die einen kritischen Gesundheitszustand des Benutzers anzeigen, an die funktionale Komponente des Kraftfahrzeugs, **dadurch gekennzeichnet, dass** die Authentisierungsinformationen eine zeitlich eindeutige Rückverfolgung eines Ursprungs des Signals erlauben.

2. Verfahren (100) gemäß Anspruch 1, ferner umfassend ein Ermitteln (114) eines Zeitfensters für eine Gültigkeit des Signals basierend auf den Authentisierungsinformationen.

3. Verfahren (100) gemäß Anspruch 2, ferner umfassend:
ein Ermitteln (118) eines Erzeugungszeitpunktes des Signals aus den Authentisierungsinformationen, wobei das Ermitteln (114) des Zeitfensters ein Festlegen (116) eines Endes des Zeitfensters auf einen Zeitpunkt spätestens eine Minute nach dem Erzeugungszeitpunkt des Signals umfasst; oder
ein Ermitteln (122) einer eindeutigen Zeichenabfolge aus den
Authentisierungsinformationen, wobei das Ermitteln (114) des Zeitfensters ein Festlegen (120) eines Endes des Zeitfensters unmittelbar nach einem Zeitpunkt umfasst, zu dem die eindeutige Zeichenabfolge erstmalig von der Kontrollvorrichtung empfangen wurde.

4. Verfahren (100) gemäß Anspruch 3, ferner umfassend ein Erzeugen (102) der eindeutigen Zeichenabfolge durch die Kontrollvorrichtung, ein Senden (104) der eindeutigen Zeichenabfolge von der Kontrollvorrichtung an das medizinische Implantat, und/oder ein Empfangen (106) der eindeutigen Zeichenabfolge.

5. Verfahren (100) gemäß einem der vorangegangenen Ansprüche, ferner umfassend ein Ermitteln (126) einer Passagierposition oder einer Fahrerposition innerhalb des Kraftfahrzeugs, an welcher sich der Benutzer befindet, basierend auf dem Signal.

6. Verfahren (100) gemäß einem der vorangegangenen Ansprüche, ferner umfassend ein Senden (128) eines Informationssignals mit Informationen über einen Fahrzeugzustand an das medizinische Implantat.

7. Verfahren (100) gemäß Anspruch 6, ferner umfassend ein Abspeichern (132) des Informationssignals.

8. Verfahren (100) gemäß einem der vorangegangenen Ansprüche, ferner umfassend ein Erzeugen (108) des Signals basierend auf einem kritischen Gesundheitszustand eines das medizinische Implantat tragenden Benutzers, wobei das Signal die Authentisierungsinformationen und die Zustandsinformationen umfasst.

9. Kontrollvorrichtung (206) für ein Kraftfahrzeug (208), umfassend:
einen Empfänger (302), welcher dazu ausgebildet ist, ein Signal (210) von einem medizinischen Implantat (202) eines Benutzers (204) des Kraftfahrzeugs (208) zu empfangen, wobei das Signal (210) Authentisierungsinformationen und Zustandsinformationen umfasst wobei die Authentisierungsinformationen eine zeitlich eindeutige Rückverfolgung eines Ursprungs des Signals erlauben;
ein Überprüfungsmodul (304), welches dazu ausgebildet ist, basierend auf den Authentisierungsinformationen eine Gültigkeit oder Ungültigkeit des empfangenen Signals (210) festzustellen; und
eine Steuereinrichtung (306), welche im Falle einer festgestellten Gültigkeit dazu ausgebildet ist, in Reaktion auf Zustandsinformationen, die einen kritischen Gesundheitszustand des Benutzers (204) anzeigen, einen Steuerbefehl (308) an eine funktionale Komponente (310) des Kraftfahrzeugs (208) bereitzustellen.

10. Kontrollvorrichtung (206) gemäß Anspruch 9, wobei das Überprüfungsmodul (304) dazu ausgebildet ist, basierend auf den Authentisierungsinformationen ein Zeitfenster zu ermitteln, und eine Gültigkeit des empfangenen Signals (210) festzustellen, wenn ein Empfangszeitpunkt des Signals (210) innerhalb des Zeitfensters liegt, oder eine Ungültigkeit des empfangenen Signals (210) festzustellen, wenn der Empfangszeitpunkt des Signals (210) außerhalb des Zeitfensters liegt.

11. Medizinisches Implantat (202), umfassend:
einen Signalerzeuger (318), welcher dazu ausgebildet ist, basierend auf einem kritischen Gesundheitszustand eines das medizinische Implantat (202) tragenden Benutzers (204) ein Signal (210) zu erzeugen, wobei das Signal (210) Authentisierungsinformationen und Zustandsinformationen umfasst, wobei die Authentisierungsinformationen eine zeitlich eindeutige Rückverfolgung eines Ursprungs des Signals erlauben; und
einen Sender (320), welcher dazu ausgebildet ist, das Signal (210) an eine Kontrollvorrichtung (206) eines Kraftfahrzeugs (208) zu senden.

12. Medizinisches Implantat (202) gemäß Anspruch 11, wobei der Signalerzeuger (318) dazu ausgebildet ist, das Signal (210) mit den Authentisierungsinformationen zu erzeugen, wobei die Authentisierungsinformationen einen Erzeugungszeitpunkt des Signals (210) und/oder eine eindeutige Zeichenabfolge (312) umfassen.

## Claims

1. A method (100) for controlling a functional component of a motor vehicle by means of a medical implant of a user of the motor vehicle, comprising:
transmitting (110) a signal comprising authentication information and state information from the medical implant to a control device of the motor vehicle;
receiving (112) the signal;
determining (124) a validity or invalidity of the received signal based on the authentication information;
providing (134) a control command, in case of a determined validity, to the functional component of the motor vehicle in response to state information indicating a critical state of health of the user, **characterized in that** the authentication information allows an origin of the signal to be traced in a temporally unique manner.

2. The method (100) according to claim 1, further comprising determining (114) a time window for a validity of the signal based on the authentication information.

3. The method (100) according to claim 2, further comprising:
ascertaining (118) a generation time of the signal from the authentication information, wherein ascertaining (114) the time window comprises setting (116) an end of the time window at a time no later than one minute after the generation time of the signal; or
ascertaining (122) a unique character sequence from the authentication information, wherein ascertaining (114) the time window comprises setting (120) an end of the time window immediately after a time at which the unique character sequence was received for the first time by the control device.

4. The method (100) according to claim 3, further comprising generating (102) the unique character sequence by means of the control device, transmitting (104) the unique character sequence from the control device to the medical implant, and/or receiving (106) the unique character sequence.

5. The method (100) according to any of the preceding claims, further comprising determining (126) a passenger position or a driver position within the motor vehicle at which the user is located based on the signal.

6. The method (100) according to any of the preceding claims, further comprising transmitting (128) an information signal comprising information about a vehicle state to the medical implant.

7. The method (100) according to claim 6, further comprising storing (132) the information signal.

8. The method (100) according to any of the preceding claims, further comprising generating (108) the signal based on a critical state of health of a user carrying the medical implant, the signal comprising the authentication information and the state information.

9. A control device (206) for a motor vehicle (208), comprising:
a receiver (302) configured to receive a signal (210) from a medical implant (202) of a user (204) of the motor vehicle (208), wherein the signal (210) comprises authentication information and state information, wherein the authentication information allows an origin of the signal to be traced in a temporally unique manner;
a checking module (304) configured to determine a validity or invalidity of the received signal (210) based on the authentication information; and
a controller (306), which, in the case of a determined validity, is configured to provide a control command (308) to a functional component (310) of the motor vehicle (208) in response to state information indicating a critical state of health of the user (204).

10. The control device (206) according to claim 9, wherein the checking module (304) is configured to ascertain a time window based on the authentication information, and to determine a validity of the received signal (210) if a reception time of the signal (210) is within the time window, or to determine an invalidity of the received signal (210) if the reception time of the signal (210) is outside the time window.

11. A medical implant (202) comprising:
a signal generator (318) configured to generate a signal (210) based on a critical state of health of a user (204) carrying the medical implant (202), wherein the signal (210) comprises authentication information and state information, wherein the authentication information allows an origin of the signal to be traced in a temporally unique manner; and
a transmitter (320) configured to transmit the signal (210) to a control device (206) of a motor vehicle (208).

12. The medical implant (202) according to claim 11, wherein the signal generator (318) is configured to generate the signal (210) with the authentication information,
wherein the authentication information comprises a generation time of the signal (210) and/or a unique character sequence (312).

## Revendications

1. Procédé (100) pour la commande d'un élément fonctionnel d'un véhicule automobile au moyen d'un implant médical d'un utilisateur du véhicule automobile, comprenant :
l'envoi (110) d'un signal, comprenant des informations d'authentification et des informations d'état, de l'implant médical vers un dispositif de contrôle du véhicule automobile ;
la réception (112) du signal ;
la constatation (124) d'une validité ou d'une non validité du signal reçu sur la base des informations d'authentification ;
la production (134) d'une instruction de commande, en cas de validité constatée, en réponse à des informations d'état indiquant un état de santé critique de l'utilisateur au niveau de l'élément fonctionnel du véhicule automobile,
**caractérisé en ce que** les informations d'authentification permettent un traçage sans ambiguïté dans le temps d'une origine du signal.

2. Procédé (100) selon la revendication 1, comprenant en outre une définition (114) d'une fenêtre temporelle pour une validité du signal sur la base des informations d'authentification.

3. Procédé (100) selon la revendication 2, comprenant en outre :
une définition (118) d'un moment de génération du signal à partir des informations d'authentification, dans lequel la définition (114) de la fenêtre temporelle comprend une constatation (116) d'une extrémité de la fenêtre temporelle à un moment au plus tard une minute après le moment de génération du signal ; ou
une définition (122) d'une séquence de caractères unique à partir des informations d'authentification, dans lequel la définition (114) de la fenêtre temporelle comprend une constatation (120) d'une extrémité de la fenêtre temporelle immédiatement après un moment auquel la séquence de caractères unique a été reçue pour la première fois par le dispositif de contrôle.

4. Procédé (100) selon la revendication 3, comprenant en outre une génération (102) de la chaîne de caractères unique par le dispositif de contrôle, un envoi (104) de la séquence de caractères unique du dispositif de contrôle à l'implant médical, et/ou une réception (106) de la séquence de caractères unique.

5. Procédé (100) selon l'une des revendications précédentes, comprenant en outre une définition (126) d'une position de passager ou d'une position de conducteur au sein du véhicule automobile au niveau de laquelle l'utilisateur est situé sur la base du signal.

6. Procédé (100) selon l'une des revendications précédentes, comprenant en outre un envoi (128) d'un signal d'information à l'implant médical comportant des informations concernant un état de véhicule.

7. Procédé (100) selon la revendication 6, comprenant en outre une mémorisation (132) du signal d'information.

8. Procédé (100) selon l'une des revendications précédentes, comprenant en outre une génération (108) du signal sur la base d'un état de santé critique d'un utilisateur portant l'implant médical, dans lequel le signal comprend les informations d'authentification et les informations d'état.

9. Dispositif de contrôle (206) pour un véhicule automobile (208), comprenant :
un récepteur (302) qui est conçu pour recevoir un signal (210) d'un implant médical (202) d'un utilisateur (204) du véhicule automobile (208), dans lequel le signal (210) comprend des informations d'authentification et des informations d'état, dans lequel les informations d'authentification permettent un traçage sans ambiguïté dans le temps d'une origine du signal ;
un module de vérification (304) qui est conçu pour constater une validité ou une non validité du signal reçu (210) sur la base des informations d'authentification ; et
une unité de commande (306) qui est conçue, en cas d'une validité constatée, pour produire une instruction de commande (308) à un élément fonctionnel (310) du véhicule automobile (208) en réponse à des informations d'état indiquant un état de santé critique de l'utilisateur (204).

10. Dispositif de contrôle (206) selon la revendication 9, dans lequel le module de vérification (304) est conçu pour définir, sur la base des informations d'authentification, une fenêtre temporelle et constater une validité du signal reçu (210) lorsqu'un moment de réception du signal (210) se trouve au sein de la fenêtre temporelle ou constater une non validité du signal reçu (210) si le moment de réception du signal (210) est en dehors de la fenêtre temporelle.

11. Implant médical (202) comprenant :
un générateur de signal (318) qui est conçu pour générer un signal (210), sur la base d'un état de santé critique d'un utilisateur (204) portant l'implant médical (202), dans lequel le signal (210) comprend des informations d'authentification et des informations d'état, dans lequel les informations d'authentification permettent un traçage sans ambiguïté dans le temps d'une origine du signal ; et
un émetteur (320) qui est conçu pour envoyer le signal (210) à un dispositif de contrôle (206) d'un véhicule automobile (208).

12. Implant médical (202) selon la revendication 11, dans lequel le générateur de signal (318) est conçu pour générer le signal (210) comportant les informations d'authentification,
dans lequel les informations d'authentification comprennent un moment de génération du signal (210) et/ou une séquence de caractères unique (312).
